# EUROPÄISCHE PATENTSCHRIFT

(11) **EP 0 721 940 B1**
(45) Veröffentlichungstag und Bekanntmachung des Hinweises auf die Patenterteilung: **22.12.1999**
(21) Anmeldenummer: 96100318.3
(22) Anmeldetag: 11.01.1996
(51) Int. Cl.: C07C 323/65, C08B 37/16, A61K 31/10

(54) **Verfahren zur Herstellung von Ajoen**
Process for the preparation of ajoene
Procédé pour la préparation de l'ajuoné

(30) Priorität: 13.01.1995 DE 19500863
(43) Veröffentlichungstag der Anmeldung: 17.07.1996
(73) Patentinhaber: Consortium für elektrochemische Industrie GmbH, 81379 München (DE)
(72) Erfinder: Dressnandt, Günter, D-81679 München (DE); Rockinger, Heinz, D-82205 Gilching (DE); Prigge, Helmut, Dr., D-82515 Wolfratshausen (DE); Treiber, Arno, Dr., D-81375 München (DE)
(74) Vertreter: Potten, Holger

(56) Entgegenhaltungen:
- DE-A- 2 948 869
- US-A- 4 665 088
- CHEMICAL ABSTRACTS, vol. 122, no. 10, 1995 Columbus, Ohio, US; abstract no. 109202f, & GAODENG XUEXIAO HUAXUE XUEBAO, Bd. 15, Nr. 8, 1994 Seiten 1172-1174, X.-Y. MA ET AL
- PLANTA MEDICA, Bd. 56, Nr. 2, 1990 Seiten 202-211, XP 000195873 B. IBERL ET AL
- CHEMICAL ABSTRACTS, vol. 108, no. 14, 4.April 1988 Columbus, Ohio, US; abstract no. 118832z, & ZHONGGUO YAOKE DAXUE XUEBAO, Bd. 18, Nr. 4, 1987 Seiten 293-6, W. YANG ET AL

## Beschreibung

Die Erfindung betrifft ein Verfahren zur Herstellung von Ajoen unter Verwendung von Cyclodextrin.

Knoblauch ist seit Jahrtausenden ein wichtiges Gewürz und Nahrungsmittel aber auch eines der bekanntesten und vielseitigsten Heilmittel in der Volksmedizin vieler Völker. Besonders häufig wird er aber auch heute noch - in letzter Zeit immer mehr - als wirksames Mittel bei einer Vielzahl von Erkrankungen eingenommen wie z.B. bei verschiedenen Infektionskrankheiten, bei einigen Stoffwechselstörungen und insbesondere bei Herz-Kreislauf-Erkrankungen. Seine Heilwirkungen wurden auch von der in den letzten Jahrzehnten intensiv betriebenen pharmakologischen Forschung bestätigt.

Aus der Chemie und Pharmakologie des Knoblauchs ist bekannt, daß der wichtigste Inhaltsstoff die Muttersubstanz Alliin [(+)-S-Allyl-L-Cysteinsulfoxid] selbst keine pharmakologische Wirksamkeit aufweist. Ferner ist bekannt, daß von den Folgeprodukten, die durch enzymatischen Abbau beim Zerkleinern des Knoblauchs entstehen, nur Allicin (Allyl-2-propenthiosulfinat) und von den Folgeprodukten die durch Umwandlung des Allicins in schwefelorganische Verbindungen entstehen nur Ajoen (4,5,9-Trithiadodeca-1,6,11-trien-9-oxid) und z.T. auch das in geringen Mengen entstehende Vinyldithiin eine hohe pharmakologische Wirksamkeit besitzen.

Alle anderen Folgeprodukte zeigen keine therapeutische Wirksamkeit.

Somit sind nach jetzigem Wissensstand Allicin und Ajoen und z.T. auch Dithiin die mit Abstand therapeutisch wichtigsten Wirksubstanzen des Knoblauchs.

Trotz ihrer nachgewiesenen therapeutischen Wirksamkeit haben diese Substanzen bisher aus folgenden Gründen keine Verwendung als standardisierte Therapiemittel gefunden:

Das durch Isolation aus Knoblauch oder durch Synthese leicht zugängliche Allicin ist sehr instabil und wandelt sich leicht und schnell in meist unwirksame Folgeprodukte wie Diallyldisulfid und Polysulfide um.

In DE-C-29 48 869 (entspricht GB 2061987) wurde deshalb versucht, Allicin unter Sauerstoffausschluß mit Cyclodextrinen zu komplexieren, um dadurch Einschlußkomplexe von Cyclodextrin und Allicin zu erhalten, die stabiler sind als das Allicin selbst. Ferner wird in DE-C-29 48 869 die Verwendung der Cyclodextrin/Allicin-Komplexe als Arzneimittelpräparate beschrieben. Wie aus den Beispielen der Anmeldung ersichtlich, ist die Stabilität der Komplexe zwar höher als die Stabilität des Allicins, aber immer noch zu niedrig als daß sie als längerfristig stabile Arzneimittelpräparate geeignet wären. Zudem sind für pharmazeutische Anwendungen reine, nicht in Komplexform vorliegende Wirkstoffe wünschenswert.

Bisher konnte kein längerfristig stabiles und somit standardisierbares Allicin-Präparat hergestellt werden.

Ajoen ist zwar sehr stabil und daher für standardisierbare Präparate geeignet, doch ist seine Gewinnung ein Problem, da es aus Allicin nur unter zeit- oder kostenaufwendigen Bedingungen in geringen Ausbeuten gewonnen werden kann.

Die Herstellung von Ajoen ist beispielsweise aus US-A-4,665,088, EP-A-0 185324 oder US-A-4,643,994 bekannt. Alle bekannten Verfahren beruhen im Prinzip auf der partiellen Umwandlung von Allicin zu Ajoen in organischen Lösungsmitteln und der anschließenden Extraktion und Isolation dieser Wirksubstanz aus der Vielzahl der erhaltenen Folgeprodukte. Nachteiligerweise besitzen all diese Umwandlungsverfahren nur eine geringe Selektivität für Ajoen und die Ausbeute an Ajoen ist sehr gering.

Chemical Abstracts, vol. 122, no. 18, abs. no. 109202f, 1995 offenbart, daß das Öl des Knoblauchs mit β-Cyclodextrin komplexiert, und dadurch Einschlußkomplexe mit β-Cyclodextrin entstehen. Aus Planta Medica, Bd 56, Seiten 202-211, 1990 ist bekannt, daß das Ajoen in dem Öl des Knoblauchs beinhaltet wird [siehe Abbildung 9].

Bisher ist ein wirtschafliches Verfahren zur Gewinnung von Ajoen nicht bekannt.

Aufgabe der vorliegenden Erfindung war es, ein wirtschaftliches Verfahren zur Gewinnung von Ajoen zur Verfügung zu stellen, welches die Nachteile des Standes der Technik vermeidet.

Die Aufgabe wird gelöst durch ein Verfahren, welches dadurch gekennzeichnet ist, daß Allicin ggf. in Wasser und/oder einem mit Wasser mischbaren Lösungsmittel gelöst, mit α-Cyclodextrin, β-Cyclodextrin, γ-Cyclodextrin oder einer beliebigen Mischung dieser Cyclodextrine zusammengegeben und zu einer wasserhaltigen Zusammensetzung verarbeitet wird, diese Zusammensetzung getrocknet wird und das entstandene Ajoen durch Dekomplexieren und Extraktion aus der getrockneten Zusammensetzung erhalten wird.

In einer bevorzugten Ausführungsform wird das getrocknete Produkt vor dem Dekomplexieren mit einem wasserfreien organischen Lösungsmittel behandelt. Durch diesen Schritt erhöht sich die Ausbeute an Ajoen erheblich.

Das erfindungsgemäße Verfahren ermöglicht es, die pharmakologisch hochwirksame Substanz Ajoen in Mengen zur Verfügung zu stellen, welche die Herstellung eines ajoenhaltigen standardisierbaren Heilmittels möglich macht.

Die Vorteile des erfindungsgemäßen Verfahrens gegenüber bekannten Methoden zur Herstellung von Ajoen sind die wesentlich höhere Ausbeute, die gute Reproduzierbarkeit und die leichte technische Durchführbarkeit des Verfahrens.

Zur Durchführung des erfindungsgemäßen Verfahrens wird wegen der bekannten Instabilität des Allicins vorzugsweise eine frische wäßrige Lösung von Allicin verwendet.

Da Allicin wegen seiner Instabilität im Handel nicht erhältlich ist, wird es vorzugsweise entweder aus Knoblauch isoliert oder durch bekannte Verfahren beispielsweise durch Oxidation des käuflich erhältlichen Diallyldisulfids hergestellt (Cavallito, C.J., Bailey, J. H., J. Am. Chem. Soc. 66, (1944), 1950 - 1951; Small, L. V. D., Bailey, J. H. Cavallito C. J., J. Am. Chem. Soc. 69, (1947), 1710 - 1713).

Zur Verbesserung der Stabilität des Allicins ist es vorteilhaft, das Allicin im erfindungsgemäßen Verfahren in einem geeigneten Lösungsmittel verdünnt einzusetzen.

Als Lösungsmittel für Allicin sind Wasser und/oder alle wassermischbaren Lösungsmittel geeignet, durch deren Einsatz die geringe Stabilität des Allicins nicht zusätzlich gefährdet wird. Beispiele für solche Lösungsmittel sind niedere (C₁ bis C₄) Alkanole oder Ketone. Vorzugsweise wird Wasser, Ethanol oder ein Ethanol/Wasser-Gemisch im Verhältnis 1:1 bis 1:99 als Lösungsmittel verwendet. Da auch die Konzentration des Allicins im Lösungsmittel die Stabilität des Allicins stark beeinflußt, wird bevorzugt mit stark verdünnten Allicin-Lösungen gearbeitet.

Besonders vorteilhaft wird Allicin im erfindungsgemäßen Verfahren deshalb als etwa 1% ige wäßrige oder wäßrig/alkoholische Lösung eingesetzt.

In dieser Form ist Allicin bei tiefen Temperaturen von vorzugsweise etwa -30°C oder tiefer längerfristig lagerfähig.

Im erfindungsgemäßen Verfahren lassen sich α-, β- oder γ-Cyclodextrin einzeln oder in beliebigen Mischungen einsetzen.

Bevorzugt geeignet werden β-Cyclodextrin und/oder γ-cyclodextrin eingesetzt.

Die Cyclodextrine können als wäßrige Lösung, als konzentrierte Suspension bzw. Paste oder in Pulverform eingesetzt werden. Als wäßrige Lösung werden sie vorzugsweise als gesättigte Lösung eingesetzt.

Cyclodextrin und Allicin werden vorzugsweise in einem Molverhältnis von 1:2 bis 1:0,5 besonders bevorzugt 1:1,2 bis 1:0,8 vermischt.

Das Vermischen des Cyclodextrins bzw. der Cyclodextrin-Lösung oder -Suspension bzw. -Paste mit der Allicinlösung wird bei 2-70°C, bevorzugt bei Anfangstemperaturen von 30-50°C und einer langsamen Abkühlung auf 2 bis etwa 20°C in üblicher Weise beispielsweise mittels Rühren durchgeführt.

Das Mischen erfolgt vorzugsweise über einen Zeitraum von 1 bis 48 h, besonders bevorzugt 2 bis 4 h.

Der Mischvorgang kann unter normalen Bedingungen (Luft) oder unter Sauerstoffauschluß in Gegenwart eines Inertgases beispielsweise Stickstoff durchgeführt werden. Er wird üblicherweise unter normalen Bedingungen, d.h. in Gegenwart von Luft durchgeführt.

Die erhaltene homogene Mischung wird als solche nach einer beliebigen in der Praxis gängigen Methode getrocknet. Beispielsweise wird die Mischung bei Raumtemperatur an der Luft oder unter Vakuum bei 20 bis 120°C bevorzugt bei 30 bis 90°C oder durch Gefriertrocknung oder durch Sprühtrocknung getrocknet.

Zur Erhöhung der Ausbeute an Ajoen ist es vorteilhaft, das erhaltene Trockenprodukt mit einem wasserfreien organischen Lösungsmittel zu behandeln.

Diesbezüglich geeignete Lösungsmittel sind vorzugsweise wasserfreie Ether, niedere Alkanole, Ketone oder Chlorkohlenwasserstoffe. Besonders geeignet ist wasserfreier Diethylether.

Vorzugsweise wird das Trockenprodukt mit dem Lösungsmittel auf einmal oder in 2 bis 3 Teilmengen im Gesamtverhältnis 1:60 bis 1:3 für einen Gesamtzeitraum von 60 bis 300 vorzugsweise 90 bis 180 min bei Raumtemperatur (ca. 20°C) oder durch Kochen unter Rückflußbedingungen vermischt und anschließend nach Abzug des Lösungsmittels (z.B. im Rotationsverdampfer unter Vakuum) in an sich bekannter Weise getrocknet. Die Trocknung kann beispielsweise im Vakuumtrockenschrank bei 30 bis 120°C erfolgen.

Das in 2 isomeren Formen (cis, trans) im Komplex gebildete Ajoen wird aus dem Trockenprodukt durch übliche Dekomplexierungsmethoden extrahiert und beispielsweise mit Hilfe eines geeigneten Lösungsmittels isoliert.

Anschließend kann es mittels an sich bekannter Trennverfahren, beispielsweise einem geeigneten chromatographischen Trennverfahren weiter aufgereinigt werden.

Alle Dekomplexierungsmethoden beruhen auf dem Lösen bzw. Suspendieren der Komplexe in viel Wasser und der Extraktion der eingeschlossenen Gastsubstanz aus der wäßrigen Phase mittels eines unpolaren Lösungsmittels sowie der Rückgewinnung aus dem Extrakt.

Die Gewinnung des Ajoens aus dem Ajoen/Cyclodextrin-Komplex erfolgt somit erfindungsgemäß durch Dekomplexieren mit Gemischen aus Wasser und mindestens einem unpolaren organischen Lösungsmittel, der Trennung der organischen und der wäßrigen Phase, und der Gewinnung des Ajoens aus der organischen Phase beispielsweise mittels Trocknung.

Geeignete organische Lösungsmittel für Ajoen sind alle bekannten Lösungsmittel für Ajoen wie beispielsweise Ether oder Chlorkohlenwasserstoffe. Bevorzugt geeignet sind Diethylether und Petrolether.

Nach Abtrennen der wäßrigen Phase wird die so erhaltene organische Phase mittels bekannter Verfahren getrocknet.

Das Ajoen kann anschließend, falls erwünscht, durch bekannte Verfahren wie z.B. durch präparative HPLC weiter aufgereinigt werden.

Der Umwandlungsgrad von Allicin in Ajoen in Gegenwart von Cyclodextrinen ist abhängig sowohl von dem verwendeten Cyclodextrin als auch von den Komplexierungs- und Nachbehandlungsbedingungen. Er ist unter den genannten bevorzugten Bedingungen und insbesondere bei Behandlung des Cyclodextrin-Komplexes mit einem wasserfreien organischen Lösungsmittel, vorzugsweise einem Ether, am höchsten.

Das im erfindungsgemäßen Verfahren durch Umwandlung des Allicins gebildete Ajoen kann sowohl in Komplexform als Cyclodextrin/Ajoen-Komplex als auch in reiner Form verwendet werden.

Als Einsatzgebiet für die Cyclodextrin/Ajoen-Komplexe oder das erfindungsgemäß hergestellte Ajoen kommt hauptsächlich der Pharmabereich in Frage. Dort läßt es sich als stabiles und daher standardisierbares Präparat besonders gegen Herz-Kreislauf-Beschwerden wie z.B. Arteriosklerose, Thromboseerscheinungen, hohen Blutdruck u.a. sowie auch gegen verschiedene bakterielle Infektionen und als Heilmittel bei einigen Organ- und Stoffwechselstörungen verwenden.

Als vorbeugendes Gesundheitsmittel kann es selbstverständlich auch im Lebensmittelbereich eingesetzt werden.

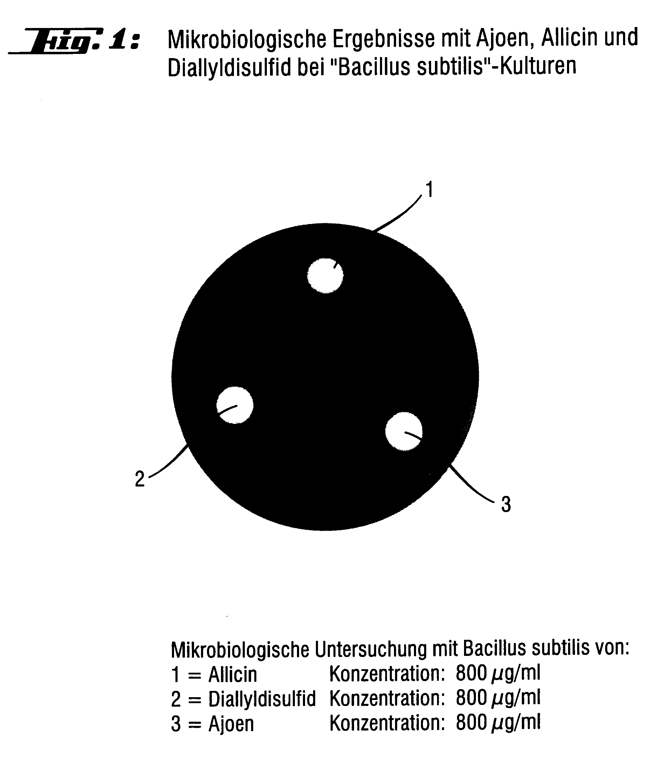

Fig. 1 zeigt die in Beispiel 8 beschriebenen Hemmhofaufnahmen. Es bedeutet:
- 1 = Allicin: Konzentration: 800 µg/ml
- 2 = Diallyldisulfid: Konzentration: 800 µg/ml
- 3 = Ajoen: Konzentration: 800 µg/ml

Die folgenden Beispiele dienen der weiteren Erläuterung der Erfindung.

Das in den Beispielen verwendete Allicin wurde nach der von Cavallito (Cavallito, C.J., Bailey, J. H., J. Am. Chem. Soc. 66, (1944), 1950 - 1951) beschriebenen Methode aus frischem Knoblauch isoliert.

Analytische Nachweise wurden in den Beispielen mittels HPLC sowie auch mittels ¹H-NMR und ¹³C-NMR durchgeführt.

### Beispiel 1

10 g α-Cyclodextrin (Wassergehalt 8,4 %) wurden als Pulver in einer Reibschale vorgelegt und portionsweise mit insgesamt 70 ml einer wäßrigen Lösung, die 0,526 g 97,3 %iges Rohallicin enthält, homogen vermischt. Die entstandene weiße Paste wurde zwei Stunden durch Weiterrühren homogenisiert, anschließend bei Raumtemperatur an der Luft getrocknet und danach zu einem feinen Pulver gemahlen. Man erhielt 10,5 g Produkt.

Dieses Zwischenprodukt wurde in 3000 ml Wasser 5 Stunden lang unter intensivem Rühren gelöst. Danach wurde die leicht trübe Lösung mit je 500 ml Diethylether dreimal extrahiert. Der vereinigte Etherextrakt wurde durch Ausfrieren entwässert (CO₂/Aceton Bad) und anschließend im Rotationsverdampfer unter Vakuum, zwecks Entfernung des Ethers, eingeengt.

Der auf diese Weise erhaltene Rückstand von 0,45 g enthält, laut ¹H-NMR-Analyse, 30,5 % Ajoen bezogen auf das eingesetzte Allicin (ca. 0,156 g) (10,3 %-Anteile cis-Ajoen, 20,2 %-Anteile trans-Ajoen).

### Beispiel 2

10 g β-Cyclodextrin (Wassergehalt 10%) wurden mit 0,87 g 79,2 %igem Allicin homogenisiert und anschließend mit 13 ml Wasser zu einer homogenen Paste verrieben. Diese wurde bei 20° C unter Vakuum getrocknet.

Man erhielt 9,67 g Trockenprodukt (Restwassergehalt = 3%), das durch Mahlen pulverisiert, mit Ether gewaschen und getrocknet wurde.

Das Ajoen wurde aus dem Komplex gewonnen durch Lösen des so erhaltenen Produkts in 3000 ml Wasser (5 stündiges intensives Rühren) und anschließende Extraktion usw. wie in Beispiel 1 beschrieben.

Der Rückstand der gesammelten Etherextrakte betrug 0,42 g. Die Ajoen-Ausbeute betrug 49,5 % bezogen auf das eingesetzte Allicin (ca. 0,34 g). Sie bestand aus 37,1 % cis- und 12,4 % trans-Ajoen.

### Beispiel 3

Es wurde wie in Beispiel 2 verfahren, jedoch mit dem Unterschied, daß das verwendete 78,8 %ige Rohallicin verdünnt mit 96 % igem Ethanol (Verhältnis 1:1) eingesetzt wurde, die Trocknung bei 30°C unter Vakuum erfolgte, das fein pulverisierte Trockenprodukt vor dem Dekomplexieren 4 h mit 100 ml Diethylether unter Rückfluß gekocht wurde, und die Dekomplexierung durch Lösen in 300 ml Wasser und 3 malige Extraktion mit je 100 ml Ether ebenfalls durch Kochen unter Rückfluß durchgeführt wurde. Der Extraktionsrückstand von 0,9 g enthielt 69,8 % Ajoen bezogen auf das eingesetzte Allicin. Er bestand aus 34,0 % cis-Ajoen und 35,8 % trans-Ajoen.

Durch Lösen in Wasser und Extraktion mit n-Heptan und Diethylether und durch präparative HPLC wurde ein 98 %iges Ajoen-Konzentrat mit gleichem cis/trans Verhältnis erhalten.

### Beispiel 4

Es wurde wie in Beispiel 3 beschrieben vorgegangen, mit dem einzigen Unterschied, daß α-Cyclodextrin verwendet wurde.

Der Extraktionsrückstand von 0,8 g enthielt 37,4 % Ajoen, bestehend aus 14,2 % cis-Ajoen und 23,2 % trans-Ajoen, bezogen auf die eingesetzte Allicinmenge.

### Beispiel 5

10 g β-Cyclodextrin (Wassergehalt = 10%) wurden unter N₂-Spülung in 200 ml Wasser bei 50°C gelöst. Unter weiterer N₂-Spülung wurde in diese Cyclodextrin-Lösung 1,41 g Allicin als 16%ige ethanolische Lösung zugetropft und 30 Minuten lang bei 50°C gerührt. Diese Mischung wurde unter Weiterrühren innerhalb von 20 Stunden auf 2 °C abgekühlt und als Ganzes gefriergetrocknet.

Es wurden 9,5 g feinkörniges Pulver erhalten, aus dem anschließend durch Dekomplexierung 1,06 g Rohprodukt mit einem Ajoengehalt von 29,2 %, bestehend aus 23,1 %-Anteilen cis-Ajoen und 6,1 %-Anteilen trans-Ajoen, bezogen auf das eingesetzte Allicin, gewonnen wurden.

Die Dekomplexierung wurde wie folgt durchgeführt: das gefriergetrocknete Pulver (9,5 g) wurde mit 600 ml Wasser, das mit HCl leicht angesäuert worden war(pH 2-3), 1,5 h unter Rückfluß gekocht. Die erhaltene Lösung wurde dreimal mit je 200 ml Ether ausgeschüttelt. Der vereinigte Etherextrakt wurde durch Ausfrieren entwässert und unter Vakuum eingeengt.

### Beispiel 6

Es wurde bis zum Gefriertrocknen wie in Beispiel 5 beschrieben vorgegangen.

Das gefriergetrocknete Pulver wurde dreimal je 30 min mit je 150 ml Diethylether geschüttelt und anschließend im Vakuumtrockenschrank getrocknet.

Die so erhaltenen 9,5 g feinkörniges Pulver wurden wie in Beispiel 5 beschrieben extrahiert. Man erhielt 1,06 g Rohprodukt mit einem Ajoengehalt von 85 % bezogen auf das eingesetzte Allicin.

### Beispiel 7

10 g γ-Cyclodextrin wurden in 43 ml Wasser bei 40°C unter Rühren gelöst. Nach Abkühlen auf 10°C wurden in diese Cyclodextrinlösung 100 ml einer 1%igen wäßrigen Allicinlösung getropft. Nach 19 Stunden unter Weiterrühren wurde der ausgefallene Niederschlag abgefiltert und getrocknet. Man erhielt 7,2 g Trockenpulver. Dieses wurde dreimal je 30 min. mit je 150 ml Diethylether geschüttelt und im Trockenschrank nachgetrocknet.

Anschließend wurde das Ajoen aus diesem Trockenpulver wie in Beispiel 1 beschrieben durch Dekomplexieren extrahiert. Im erhaltenen Extraktionsrückstand von 0,9 g wurden 0,457 g Ajoen bestimmt. Dies entspricht einer Ajoen-Ausbeute von 45,7 %.

### Beispiel 8

Getestet wurde die Hemmwirkung von gemäß Beispiel 3 hergestelltem Ajoen sowie von Allicin und Diallyldisulfid auf das Wachstum von Testbakterien (Bacillus subtilis) auf einem hiermit infizierten Nährboden (LB-Medium).

Dazu wurden auf eine sterile Petrischale von 10 cm Durchmesser zuerst 20 ml einer Nährbodenlösung (L.B. -Medium) ausgegossen. Nach Erstarren der ca. 1 cm dicken Schicht wurde darüber eine ca. 1 mm dicke Bakterien-Kultur (Bacillus subtilis) geschichtet (Lösung a + b) und darauf ein mit 40 µl der zu untersuchenden Substanz lösung getränktes Filterplättchen (ca. 10 mm Durchmesser) mit einer sterilen Pinzette gelegt. Man ließ die Lösung ca. 2 Stunden auf die Bakterienschicht im Kühlschrank (5°C) einwirken und bebrütete anschließend ca. 16 Stunden bei 28°C. Der um die Filterplättchen gebildete Hemmhof wurde ausgemessen (Durchmesser oder Breite) und photographiert. Er stellt ein Kriterium für die antibakterielle Wirksamkeit der getesteten Substanz dar.

| Lösung a = Nährlösung (LB-flüssig) | |
|---|---|
| 10 g Trypton | |
| 5 g Hefeextrakt | |
| 5 g NaCl | wurden mit Wasser auf aufgefüllt und sterilisiert |
| 1 l | |

| Lösung b = LB-Weichagar | |
|---|---|
| 5 g Trypton | |
| 2,5 g Hefeextrakt | |
| 2,5 g NaCl | |
| 3,0 g Agar | wurden mit Wasser auf aufgefüllt und sterilisiert |
| 1 l | |

Die Filterpapierplättchen wurden ebenso 20 Min. bei 120° C sterilisiert.

### Herstellung der Bacillus-subtilis-Kultur

1 ml Bacillus-subtilis-Anzucht-Lösung wird in einen Erlenmeyer-Kolben mit 10 ml Nährlösung (Lsg. a) pipettiert und die Kultur über Nacht bei 37°C und 260 UPM im Wasserbadrüttler angezüchtet.

0,1 ml dieser Bacillus-subtilis-Lösung werden zu 10 ml des durch schwache Erwärmung flüssig gemachten Weichagar (Lsg. b) gegeben, durch kurzes Schwenken vermischt und auf den Nährboden in der Schale geschichtet. Diese Menge reicht für 1 Petrischale.

Die in Fig. 1 dargestellten Hemmhofaufnahmen zeigen, daß bei Wirkstoff-Konzentrationen von 800 µg/ml mit Ajoen gleich gute antibakterielle Hemmwirkungen erreicht werden wie mit Allicin, während bei der Verwendung von Diallyldisulfid, der Hauptkomponente des "Knoblauchöls", überhaupt keine Wirkung erzielt wird.

## Patentansprüche

1. Verfahren zur Gewinnung von Ajoen, dadurch gekennzeichnet, daß Allicin ggf. in Wasser und/oder einem mit Wasser mischbaren Lösungsmittel gelöst, mit α-Cyclodextrin, β-Cyclodextrin, γ-Cyclodextrin oder einer beliebigen Mischung dieser Cyclodextrine zusammengegeben und zu einer wasserhaltigen Zusammensetzung verarbeitet wird, diese Zusammensetzung getrocknet wird und das entstandene Ajoen durch Dekomplexieren und Extraktion aus der getrockneten Zusammensetzung erhalten wird.

2. Verfahren gemäß Anspruch 1, dadurch gekennzeichnet, daß die getrocknete Zusammensetzung vor dem Dekomplexieren mit einem wasserfreien organischen Lösungsmittel behandelt wird.

3. Verfahren gemäß Anspruch 2, dadurch gekennzeichnet, daß als wasserfreies organisches Lösungsmittel Ether eingesetzt wird.

4. Verfahren gemäß Anspruch 3, dadurch gekennzeichnet, daß als Ether Diethylether eingesetzt wird.

5. Verfahren gemäß einem oder mehreren der Ansprüche 1 bis 4, dadurch gekennzeichnet, daß das Allicin in Form einer etwa 1% igen wäβrigen Lösung eingesetzt wird.

6. Verfahren gemäß einem oder mehreren der Ansprüche 1 bis 5, dadurch gekennzeichnet, daß als Cyclodextrin β-Cyclodextrin und/oder γ-Cyclodextrin eingesetzt wird.

7. Komplexe von α-, β- oder γ-cyclodextrin mit Ajoen.

## Claims

1. Process for obtaining ajoene, characterized in that allicin is dissolved where appropriate in water and/or a water-miscible solvent, mixed with α-cyclodextrin, β-cyclodextrin, γ-cyclodextrin or any desired mixture of these cyclodextrins, and processed to a water-containing composition, this composition is dried, and the resulting ajoene obtained by decomplexation and extraction from the dried composition.

2. Process according to Claim 1, characterized in that the dried composition is treated before the decomplexation with an anhydrous organic solvent.

3. Process according to Claim 2, characterized in that ether is employed as anhydrous organic solvent.

4. Process according to Claim 3, characterized in that diethyl ether is employed as ether.

5. Process according to one or more of Claims 1 to 4, characterized in that allicin is employed in the form of an approximately 1% strength aqueous solution.

6. Process according to one or more of Claims 1 to 5, characterized in that β-cyclodextrin and/or γ-cyclodextrin is employed as cyclodextrin.

## Revendications

1. Procédé d'obtention d'ajoène, caractérisé en ce que l'on dissout de l'allicine éventuellement dans de l'eau et/ou un solvant miscible à l'eau, on mélange avec une α-cyclodextrine, une β-cyclodextrine, une γ-cyclodextrine ou un mélange quelconque de ces cyclodextrines, et on met en oeuvre sous forme d'une composition contenant de l'eau, on sèche cette composition et on obtient l'ajoène résultant par décomplexation et extraction à partir de la composition séchée.

2. Procédé selon la revendication 1, caractérisé en ce que la composition séchée est traitée avec un solvant organique anhydre avant la décomplexation.

3. Procédé selon la revendication 2, caractérisé en ce que l'on utilise un éther en tant que solvant organique anhydre.

4. Procédé selon la revendication 3, caractérisé en ce que l'on utilise l'éther diéthylique en tant qu'éther.

5. Procédé selon l'une ou plusieurs des revendications 1 à 4, caractérisé en ce que l'on utilise l'allicine sous forme d'une solution aqueuse à environ 1%.

6. Procédé selon l'une ou plusieurs des revendications 1 à 5, caractérisé en ce que l'on utilise la β-cyclodextrine et/ou la γ-cyclodextrine en tant que cyclodextrine.
